(19) Europäisches Patentamt
European Patent Office
Office européen des brevets

(11) Veröffentlichungsnummer: **0 252 363 B1**

(12) # EUROPÄISCHE PATENTSCHRIFT

(45) Veröffentlichungstag der Patentschrift: **02.01.91 Patentblatt 91/01**

(51) Int. Cl.$^5$: **C07D 207/38**

(21) Anmeldenummer: **87108974.4**

(22) Anmeldetag: **23.06.87**

(54) **4-Benzyloxy-3-pyrrolin-2-on, dessen Herstellung und Verwendung zur Synthese von Tetramsäure.**

(30) Priorität: **26.06.86 CH 2566/86**

(43) Veröffentlichungstag der Anmeldung: **13.01.88 Patentblatt 88/02**

(45) Bekanntmachung des Hinweises auf die Patenterteilung: **02.01.91 Patentblatt 91/01**

(84) Benannte Vertragsstaaten: **AT BE CH DE ES FR GB IT LI LU NL SE**

(56) Entgegenhaltungen:
**DE-A- 2 328 277**
**US-A- 3 299 095**
**JOURNAL OF THE CHEMICAL SOCIETY - PERKIN TRANSACTIONS I, 1973, Seiten 2907-2910; G. LOWE et al.: "Synthesis of a beta-lactam related to the Cephalosporins"**

(73) Patentinhaber: **LONZA AG**
**Gampel/Wallis (CH)**

(72) Erfinder: **Meul, Thomas, Dr.**
**Balfrinstrasse 21**
**Visp (Kanton Wallis) (CH)**

(74) Vertreter: **Weinhold, Peter, Dr. et al**
**Patentanwälte Dr. V. Schmied-Kowarzik Dipl.-Ing. G. Dannenberg Dr. P. Weinhold Dr. D. Gudel Dipl.-Ing. S. Schubert Dr. P. Barz**
**Siegfriedstrasse 8**
**D-8000 München 40 (DE)**

EP 0 252 363 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Tetramsäure ist ein wertvolles Ausgangsprodukt zur Herstellung von ß-Lactamen, die ihrerseits für die Herstellung wirksamer Antibiotika eingesetzt werden. (G. Lowe, J. chem. Soc., Perkin Trans. I, 1973, 2907.)

Bisher hat es an vorteilhaften Verfahren gemangelt, Tetramsäure herzustellen.
So ist aus Lowe, J. chem. Soc., Perkin Trans. I, 1973, 2909 bekannt, Malonsäuremonoethylester mit Glycinethylester in Gegenwart von Dicyclohexylcarbodiimid zum N-Ethoxy-(carbonyl-acetyl)-glycinethylester umzusetzen, in einer weiteren Stufe mit einer Base zum 2,4-Dioxopyrrolidin-3-carbonsäuremethylester ringzuschliessen und schliesslich zur Tetramsäure zu decarboxylieren.

Der wesentliche Nachteil dieses Verfahrens ist aber die notwendige, extrem hohe Verdünnung der Reaktionslösung in der letzten Stufe (3,39 g auf 2,5 Liter = 0,1%-ige Lösung), die ein wirtschaftliches Verfahren in grösserem Massstab ausschliesst.

Aufgabe war es daher, ein Verfahren zu finden, das ohne diese Nachteile auskommt.

Durch die neue einfach zugängliche Zwischenstufe - das 4-Benzyloxy-3-pyrrolin-2-on - konnte die Aufgabe auf überraschend einfache Weise gelöst werden.

Diese Verbindung kann erfindungsgemäß durch säurekatalysierte Umesterung von 4-Alkoxy-3-pyrrolin-2-onen mit Benzylalkohol hergestellt Werden.

Ebenso einfach sind die 4-Alkoxy-3-pyrrolin-2-one nach einem bekannten Verfahren aus 4-Halogenacetessigestern herstellbar EP-A-216 324.
Bevorzugt wird als Ausgangsprodukt das 4-Methoxy-3-pyrrolin-2-on eingesetzt.
Als Säuren für die Umesterung finden zweckmässig wasserfreie anorganische Säuren, wie z.B. Schwefelsäure oder Sulfonsäuren, wie z.B. Methansulfonsäure Anwendung. Bevorzugt wird Methansulfonsäure eingesetzt.
Diese werden in katalytischen Mengen von zweckmässig 5 bis 20 Mol%, vorzugsweise 5 bis 10 Mol% eingesetzt.

Den Reaktanden Benzylalkohol setzt man zweckmässig in einem Ueberschuss von 50 bis 200%, bezogen auf das eingesetzte 4-Alkoxy-3-pyrrolin-2-on, ein.

Vorteilhaft fungiert Benzylalkohol gleich selber als Lösungsmittel.

Die Umsetzung findet vorteilhaft bei Temperaturen zwischen 60 und 100°C, bevorzugt zwischen 70 und 90°C statt.

Man arbeitet bevorzugt unter vermindertem Druck, insbesondere zwischen 1 bis 50 mbar, um die abgespaltenen niedrigen Alkohole aus dem Gleichgewicht zu entfernen.
Nach einer Reaktionszeit von ungefähr 20 bis 30 Stunden kann das 4-Benzyloxy-3-pyrrolin-2-on auf übliche Weise, z.B. durch azeotropes Abdampfen des überschüssigen Benzylalkohols und gegebenenfalls durch anschliessende Kristallisation aufgearbeitet werden.

Erfindungsgemäß kann das erhaltene Produkt auf einfache Weise durch katalytische Hydrogenolyse in die Tetramsäure überführt werden.
Besonders geeigneter Katalysator dafür ist Palladium, in einer Menge von zweckmässig 5 bis 20% aufgebracht auf Kohle.

Es wird mit Vorteil in wasserfreien, polaren, aprotischen Lösungsmitteln, bevorzugt in Tetrahydrofuran, Dioxan oder Dimethylformamid als Lösungsmittel gearbeitet.

Die Reaktionstemperaturen bewegen sich zweckmässig zwischen 0 und 40°C, die Reaktionsdrücke zwischen 1 und 20 bar.

Nach üblicher Aufarbeitung, die abhängig von Druck und Temperatur nach 5 bis 10 Stunden erfolgen kann, wird die Tetramsäure in nahezu quantitativer Ausbeute erhalten.

### Beispiel

#### Herstellung von 4-Benzyloxy-3-pyrrolin-2-on

5,7 g (50 mMol) 4-Methoxy-3-pyrrolin-2-on und 10,8 g (100 mMol) Benzylalkohol wurden mit 0,4 g (4 mMol) Methansulfonsäure versetzt und 24 Stunden bei 80°C und 20 mbar gerührt. Anschliessend wurde die Reaktionslösung mit 50 ml Eiswasser und 100 ml Methylenchlorid versetzt und mit 4 ml gesättigter $NaHCO_3$-Lösung neutralisiert. Die wässrige Phase wurde noch zweimal mit je 50 ml Methylenchlorid extrahiert. Nach Trocknen der organischen Phase über $Na_2SO_4$ und Abdestillieren des Lösungsmittels wurde der Rückstand mit 150 ml Eiswasser versetzt, auf 100°C erhitzt und 100 ml Wasser-Benzylalkohol azeotrop abdestilliert. Die beim Abkühlen ausgefallenen Kristalle wurden aus 50 ml Toluol heiss umkristallisiert.

Man erhielt 6,7 g weisses, kristallines Produkt mit einem Schmelzpunkt von 147 bis 148°C.
NMR : (300 MHz, DMSO-$d_6$) δ in ppm

7,38 (m, 5H), 6,20 (br. s, 1H), 5,16 (s, 1H), 4,98 (s, 2H), 3,98 (s, 2H).
MS (70 eV) : 189 ($M^+$, 40), 172 (18), 132 (51), 91 (100).

Herstellung von 2,4-Dioxopyrrolidin (Tetramsäure)

1,0 g (5,3 mMol) 4-Benzyloxy-3-pyrrolin-2-on wurde in 50 ml Tetrahydrofuran gelöst und in Gegenwart von 100 mg Pd/C 5% 6 Stunden bei Raumtemperatur und 10 bar hydrogenolysiert. Anschliessend wurde vom Katalysator abfiltriert und das Filtrat am Rotationsverdampfer zur Trockene eingeengt.
Man erhielt 500 mg weisses, kristallines Produkt mit einem Schmelzpunkt von 120°C (über 120°C wurde das Produkt wieder fest).
NMR : (300 MHz, DMSO-$d_6$) δ in ppm
Enolform : 11,28 (s, 1H), 7,11 (br. s, 1H), 4,76 (s, 1H), 3,74 (s, 2H).
Ketoform : 8,25 (br. s, 1H), 3,78 (s, 2H), 2,93 (s, 2H).
MS (70 eV) : 99 ($M^+$, 32), 71 (78), 43 (58), 42 (100).

## Ansprüche

**Patentansprüche für die Vertragstaaten : HI, BE, CH, DE, FR, GB, IT, LI, LU, NL, SE**

1. 4-Benzyloxy-3-pyrrolin-2-on der Formel

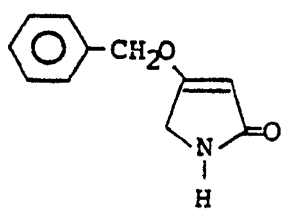

1

2. Verfahren zur Herstellung der Verbindung nach Patentanspruch 1, dadurch gekennzeichnet, dass man 4-Alkoxy-3-pyrrolin-2-on sauer mit Benzylalkohol umsetzt.

3. Verfahren nach Patentanspruch 2, dadurch gekennzeichnet, dass man als Säuren Sulfonsäuren oder wasserfreie anorganische Säuren verwendet.

4. Verfahren nach Patentansprüchen 2 und 3, dadurch gekennzeichnet, dass die Reaktion bei Temperaturen von 60 bis 100°C durchgeführt wird.

5. Verfahren nach Patentansprüchen 2, 3 und 4, dadurch gekennzeichnet, dass die Reaktion unter vermindertem Druck durchgeführt wird.

6. Verwendung der Verbindung nach Patentanspruch 1 zur Herstellung von Tetramsäure der Formel

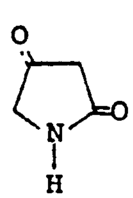

2

dadurch gekennzeichnet, dass man diese einer katalytischen Hydrogenolyse unterzieht.

7. Verwendung nach Patentanspruch 6, dadurch gekennzeichnet, dass als Katalysator Palladium verwendet wird.

8. Verwendung nach Patentansprüchen 6 und 7, dadurch gekennzeichnet, dass bei Temperaturen von

0 bis 40°C und Drücken von 1 bis 20 bar gearbeitet wird.

**Ansprüche**

**Patentansprüche für den Vertragstaat : ES**

1. Verfahren zur Herstellung von 4-Benzyloxy-3-pyrrolin2-on der Formel

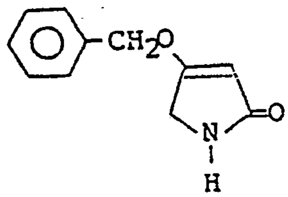

1

dadurch gekennzeichnet, daß man 4-Alkoxy-3-pyrrolin2-on sauer mit Benzylalkohol umsetzt.

2. Verfahren nach Patentanspruch 1, dadurch gekennzeichnet, dass man als Säuren Sulfonsäuren oder wasserfreie anorganische Säuren verwendet.

3. Verfahren nach Patentansprüchen 1 und 2, dadurch gekennzeichnet, dass die Reaktion bei Temperaturen von 60 bis 100°C durchgeführt wird.

4. Verfahren nach Patentansprüchen 1, 2 und 3, dadurch gekennzeichnet, dass die Reaktion unter vermindertem Druck durchgeführt wird.

5. Verfahren zur Herstellung von Tetramsäure der Formel

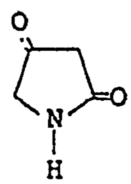

2

dadurch gekennzeichnet, daß man 4-Benzyloxy-3-pyrrolin2-on der Formel

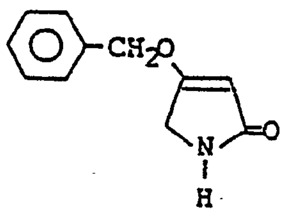

einer katalytischen Hydrogenolyse unterzieht.

6. Verfahren nach Patentanspruch 5, dadurch gekennzeichnet, daß als Katalysator Palladium verwendet wird.

7. Verfahren nach Patentansprüchen 5 und 6, dadurch gekennzeichnet, daß bei Temperaturen von 0 bis 40°C und Drücken von 1 bis 20 bar gearbeitet wird.

**Claims**

**Claims for the Contracting States : AT, BE, CH, DE, FR, GB, IT, LI, LU, NL, SE**

1. 4-Benzyloxy-3-pyrroline-2-one of the formula

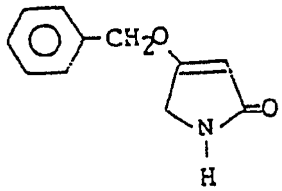

1

2. Process for the preparation of the compound according to patent claim 1, characterized in that 4-alkoxy-3-pyrroline-2-one is reacted in the presence of acids with benzyl alcohol.

3. Process according to patent claim 2, characterized in that the acids are sulfonic acids or anhydrous inorganic acids.

4. Process according to patent claims 2 and 3, characterized in that the reaction is carried out at temperatures of 60 to 100°C.

5. Process according to patent claims 2, 3 and 4, characterized in that the reaction is carried out under reduced pressure.

6. The use of the compound according to patent claim 1 in the preparation of tetramic acid of the formula

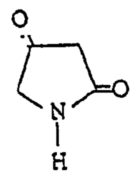

2

characterized in that the same is subjected to a catalytic hydrogenolysis.

7. The use according to patent claim 6, characterized in that the catalyst is palladium.

8. The use according to patent claims 6 and 7, characterized in that one operates at temperatures of 0 to 40°C and at pressures of 1 to 20 bar.

**Claims for the Contracting State : ES**

1. Process for the preparation of 4-benzyloxy-3-pyrroline-2-one of the formula

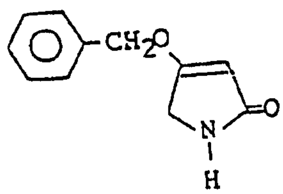

1

characterized in that 4-alkoxy-3-pyrroline-2-one is reacted in the presence of acids with benzyl alcohol.

2. Process according to patent claims 1, characterized in that the acids are sulfonic acids or anhydrous inorganic acids.

3. Process according to patent claims 1 and 2, characterized in that the reaction is carried out at temperatures of 60 to 100°C.

4. Process according to patent claims 1, 2 and 3, characterized in that the reaction is carried out under reduced pressure.

5. Process for the preparation of tetramic acid of the formula

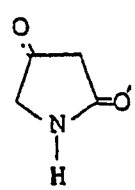

2

characterized in that 4-benzyloxy-3-pyrroline-2-one of the formula

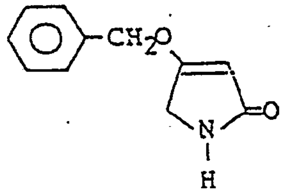

is subjected to a catalytic hydrogenolysis.

6. Process according to patent claim 5, characterized in that the catalyst is palladium.

7. Process according to patent claims 5 and 6, characterized in that one operates at temperatures of 0 to 40°C and at pressures of 1 to 20 bar.

## Revendications

### Revendications pour les Etats contractants :AT, BE, CH, DE, FR, GB, IT, LI, LU, NL, SE

1. 4-Benzyloxy-3-pyrroline-2-one de formule

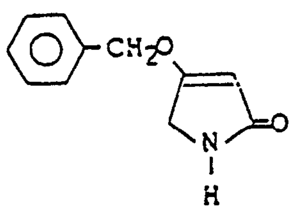

1

2. Procédé pour la fabrication du composé selon la revendication 1, caractérisé en ce que l'on fait réagir une 4-alcoxy-3pyrroline-2-one, en milieu acide, avec l'alcool benzylique.

3. Procédé selon la revendication 2, caractérisé en ce que l'on utilise comme acides des acides sulfoniques ou des acides non organiques, anhydres.

4. Procédé selon les revendications 2 et 3, caractérisé en ce que la réaction est effectuée à des températures de 60 à 100°C

5. Procédé selon les revendications 2, 3 et 4, caractérisé en ce que la réaction est effectuée sous pression réduite.

6. Utilisation du composé selon la revendication 1 pour la fabrication de la pyrrolidinedione-2,4 de formule

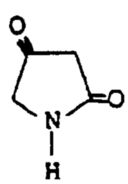

2,

caractérisée en ce que l'on soumet celle-ci à une hydrogènolyse catalytique.

7. Utilisation selon la revendication 6, caractérisée en ce que l'on utilise le palladium comme catalyseur

8. Utilisation selon les revendications 6 et 7, caractérisée en ce que l'on travaille à des températures de 0 à 40°C et sous des pressions de 1 à 20 bars.

### Revendications pour l'Etat contractant : ES

1. Procédé pour la fabrication de la 4-benzyloxy-3-pyrroline-2-one de formule

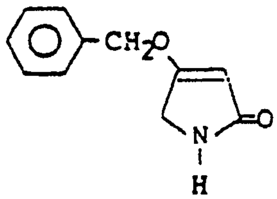

1,

caractérisé en ce que l'on fait réagir une 4-alcoxy-3-pyrroline2-one, en milieu acide, avec l'alcool benzylique.

2. Procédé selon la revendication 1, caractérisé en ce l'on utilise comme acides, des acides sulfoniques ou des acides non organiques anhydres.

3. Procédé selon les revendications 1 et 2, caractérisé en ce que la réaction est effectuée à des températures de 60 à 100°C

4 Procédé selon les revendications 1, 2 et 3, caractérisé en ce que la réaction est effectuée sous pression réduite

5. Procédé pour la fabrication de la pyrrolidinedione-2,4 de formule

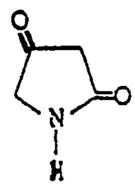

2,

caractérisé en ce qu'on soumet la 4-benzyloxy-3-pyrroline-2-one de formule

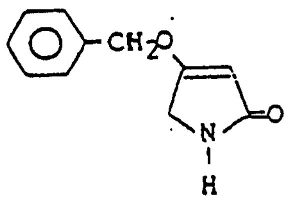

à une hydrogènolyse catalytique

6. Procédé selon la revendication 5, caractérisé en ce que l'on utilise le palladium comme catalyseur.

7. Procédé selon les revendications 5 et 6, caractérisé en ce que l'on travaille à des températures de 0 à 40°C et sous des pressions de 1 à 20 bars.